Europäisches Patentamt

European Patent Office

Office européen des brevets

(11) Veröffentlichungsnummer: **0 291 843 B1**

(12) **EUROPÄISCHE PATENTSCHRIFT**

(45) Veröffentlichungstag der Patentschrift: **22.02.95**

(21) Anmeldenummer: **88107580.8**

(22) Anmeldetag: **11.05.88**

Die Akte enthält technische Angaben, die nach dem Eingang der Anmeldung eingereicht wurden und die nicht in dieser Patentschrift enthalten sind.

(51) Int. Cl.⁶: **G01N 33/52**, G01N 33/72, A61B 10/00, //G01N33/50, G01N33/68

(54) Testkit zur Bestimmung eines Analyten im Stuhl.

(30) Priorität: **20.05.87 DE 3716891**

(43) Veröffentlichungstag der Anmeldung:
**23.11.88 Patentblatt  88/47**

(45) Bekanntmachung des Hinweises auf die Patenterteilung:
**22.02.95 Patentblatt  95/08**

(84) Benannte Vertragsstaaten:
**AT BE CH DE ES FR GB GR IT LI LU NL SE**

(56) Entgegenhaltungen:
**EP-A- 0 070 366**
**EP-A- 0 117 689**
**EP-A- 0 279 574**
**FR-A- 2 514 511**
**US-A- 3 902 847**

(73) Patentinhaber: **BOEHRINGER MANNHEIM GMBH**

**D-68298 Mannheim (DE)**

(72) Erfinder: **Steinbiss, Joachim, Dr. Dipl.-Biol.**
**Alexanderstrasse 21a**
**D-6143 Lorsch (DE)**
Erfinder: **Trasch, Heinz-Friedrich, Dr. rer. nat. Dipl.-Chem.**
**Wissmannstrasse 14**
**D-6700 Ludwigshafen (DE)**

(74) Vertreter: **Pfeifer, Hans-Peter, Dr.rer.nat.**
**Patentanwalt**
**Nowackanlage 15**
**D-76137 Karlsruhe (DE)**

**Beschreibung**

Die Erfindung betrifft einen Testkit zur Bestimmung eines Analyten in einer pastösen, feste Bestandteile enthalten den Probe. Außerdem richtet sich die Erfindung auf ein Verfahren zur Durchführung einer solchen Bestimmung mit einem solchen Testkit.

Das wichtigste Anwendungsgebiet der Erfindung ist die Analyse von Stuhl. Andere Probenmaterialien, für deren Analyse die Erfindung vorteilhaft eingesetzt werden kann, sind Homogenisate tierischer oder menschlicher Gewebeproben, galenische Aufschlemmungen im Rahmen der pharmazeutischen Analytik und Erdproben. Allgemein ist die Erfindung für die Analyse pastöser, streichfähiger Proben, welche feste Bestandteile enthalten, besonders geeignet. Im folgenden wird der Einfachheit halber, jedoch ohne Beschränkung der Allgemeinheit, nur auf die Analyse von Stuhl eingegangen.

Zur Bestimmung der Anwesenheit oder Konzentration eines Analyten in Stuhlproben werden häufig Testkits verwendet. Allgemein bezeichnet man damit Kombinationen von für die Analyse notwendigen Reagenzien und Hilfsmitteln.

Von besonderer praktischer Bedeutung ist die Untersuchung des Stuhls auf darin enthaltenes okkultes Blut. Im Rahmen der Maßnahmen zur Früherkennung von Karzinomen und Präkanzerosen des Colons und Rektums stellt der Nachweis von okkultem Blut die anerkannte Methode dar.

Bei derartigen Massenuntersuchungen ist eine einfache Handhabung des Testkits besonders wichtig. Üblicherweise werden derzeit sogenannte Testbriefchen verwendet, welche, umrahmt von einer Umhüllung aus Karton, eine Probenschicht haben, die üblicherweise aus Guajak-imprägniertem Papier besteht. Nach dem Auftragen der Probe auf ein Probenfeld auf der Oberfläche der Probenschicht wird das Briefchen auf der Vorderseite geschlossen.

Die Auswertung wird üblicherweise beim Arzt durchgeführt, wobei eine Klappe auf der Rückseite des Briefchens geöffnet und auf die Rückseite des Guajak-imprägnierten Papiers eine Entwicklungslösung aufgegeben wird, welche Wasserstoffperoxid enthält. Bei Anwesenheit von Blut im Stuhl ergibt sich dabei ein Farbumschlag.

Im Hinblick darauf, daß der Blutgehalt des Stuhls häufig nicht homogen ist, werden im allgemeinen Doppelbriefchen verwendet, auf die jeweils gleichzeitig zwei Proben von verschiedenen Stellen des Stuhls aufgetragen werden. Außerdem ist es üblich, in einem Testkit jeweils drei derartige Briefchen zu verwenden, mit denen an drei aufeinanderfolgenden Tagen der Stuhl untersucht wird. Sofern nur eine dieser Proben positiv ist, sind weitere Untersuchungsmaßnahmen angezeigt.

Diese Testbriefchen zur Untersuchung des Stuhls auf okkultes Blut erfüllen im wesentlichen die Anforderungen an die Handhabung eines solchen Tests. Sowohl die beim Patienten als auch beim Arzt durchzuführenden Handhabungsschritte sind relativ einfach. Der Test läuft ausreichend schnell ab. Die Auswertung kann ohne apparative Hilfe erfolgen, und die Herstellung ist verhältnismäßig kostengünstig.

Diese einfache Handhabung wurde bisher aber nur beim Stuhlbluttest mit Hilfe von Guajakpapieren erreicht. Dieser hat jedoch erhebliche diagnostische Nachteile. Insbesondere ist er einerseits nicht ausreichend spezifisch und andererseits in vielen Fällen nicht ausreichend empfindlich.

Die mangelnde Spezifität ist darauf zurückzuführen, daß die auf der pseudoperoxidatischen Reaktion des Hämoglobins basierende Nachweismethode auch auf andere Stuhlbestandteile reagiert, wie beispielsweise mit der Nahrung zugeführtes tierisches Blut. Um falsch-positive Befunde zu vermeiden, muß der Test daher unter kontrollierter Diät durchgeführt werden.

Außerdem wird bewußt mit einer relativ hohen Empfindlichkeitsschwelle gearbeitet. Dies hat wiederum den Nachteil, daß die Gefahr besteht, geringe Blutkonzentrationen, die dennoch klinisch relevant sind, zu übersehen, also falsch-negative Befunde zu erhalten.

Wegen dieser Nachteile hat es bereits zahlreiche Versuche gegeben, andere Testprinzipien zur Analyse von Stuhlbestandteilen und insbesondere zum Nachweis von okkultem Blut einzusetzen. In neuerer Zeit sind insbesondere immunologische Verfahren vorgeschlagen worden, bei denen für das Hämoglobin spezifische immunologische Bindungspartner (Hb-Antikörper) verwendet werden. Derartige Testkits sind jedoch aufwendig in der Handhabung.

Insbesondere erfordern die bekannten immunologischen Untersuchungen des Stuhls üblicherweise aufwendige Probenvorbereitungsmaßnahmen. Üblicherweise muß die Probe homogenisiert, danach mit einer Flüssigkeit gemischt und schließlich zentrifugiert werden. Erst der so erzeugte reine Überstand kann als Probenflüssigkeit eingesetzt werden.

Unter dem Markennamen HEMOLEX ist ein Testkit zur immunologischen Bestimmung von Hämoglobin im Stuhl bekannt, der die Zentrifugation vermeidet. Dennoch ist der Vorgang sehr aufwendig. Beispielsweise muß der Stuhl unmittelbar im frischen Zustand homogenisiert und in einer speziellen Lösung aufgenommen werden. Soweit dies nicht möglich ist, muß gekühlt werden.

Aus der DE-A 34 02 938 ist eine Diagnosehilfe für die immunologische Bestimmung von Hämoglobin im Stuhl bekannt, bei der ein fester stabförmiger Träger, welcher mit dem Antikörper beschichtet ist, unmittelbar in den Stuhl eingetaucht wird. Die weitere Auswertung erfordert mehrere naßchemische Schritte, insbesondere Waschungen, und ist daher aufwendig.

Aus der EP-B1-32 782 ist ein Testkit bekannt, bei dem ein vorzugsweise aus Kunststoff hergestellter Formkörper verwendet wird, in dessen Probenauftragungsbereich sich ein Guajakimprägniertes Filterpapier befindet. Dieses dient hier aber nicht nur zum Nachweis, sondern zugleich zur Filterung der Probe. Während diese auf die Vorderseite des Papiers aufgegeben wird, befindet sich auf der Rückseite ein unbehandeltes Filterpapier. Durch den besonderen Aufbau des Formkörpers ist es möglich, die Probe zusammenzupressen, wobei das Hämoglobin aus der Probe zusammen mit flüssigen Probenbestandteilen durch das Guajakpapier in das unbehandelte Filterpapier übertritt. Zur Auswertung wird das Filterpapier entnommen und naßchemisch ausgewertet.

Aufgabe der vorliegenden Erfindung ist es, einen Testkit und ein Verfahren zur Analyse von Stuhl zur Verfügung zu stellen, bei dem aufwendige Handhabungsschritte, insbeson-für die Probenvorbereitung, vermieden werden.

Die Aufgabe wird durch einen Testkit gemäß Anspruch 1 gelöst.

Das erfindungsgemäße Verfahren weist die Merkmale von Anspruch 10 auf.

Mit der erfindungsgemäßen Vorrichtung und dem entsprechenden Verfahren wird eine Probengewinnung aus einer Stuhlprobe auf sehr einfach Weise möglich. Überraschenderweise ergibt sich, daß das Eluationsmittel einerseits in der Lage ist, den Analyten in weitgehend reproduzierbarer Art und Weise aus der Stuhlprobe zu lösen und in den Nachweisbereich zu transportieren, während andererseits praktisch keine festen Stuhlbestandteile aus der Probenschicht in den räumlich getrennten Eluationsaufnahmebereich gelangen.

Als kapillaraktiv im Sinne der Erfindung ist jedes Schichtmaterial anzusehen, welches aufgrund von kapillarformenden Strukturen die Eigenschaft hat, das flüssige Eluationsmittel in den Kapillaren aufzusaugen. Außerdem muß die Oberfläche der kapillarförmigen Strukturen aus einem Material bestehen, welches von der Eluationsflüssigkeit benetzt wird. Im übrigen muß das Material selbstverständlich beständig gegenüber der vorzugsweise wässrigen Eluationslösung sein und darf die Reaktion nicht ungünstig beeinflussen.

Als Materialien kommen sowohl Naturstoffe als auch hydrophile Kunststoffe in Frage, die zur Erzeugung der kapillarformenden Strukturen als Papiere, Vliese, Gewebe oder entsprechend poröse oder strukturierte Folien verarbeitet sind.

Die Chromatographie einer Flüssigkeit in einem kapillaraktiven Material wird auch in der US-Patentschrift 3 902 847 eingesetzt, welche einen Test zur Früherkennung der Mucoviscidosis, betrifft. Der Test basiert darauf, daß in dem von neugeborenen Kindern abgeschiedenen Mekonium die Albumin-Konzentration bestimmt wird. Hierzu wird ein Albumin-Testpapier verwendet, das auf einem Plastikstreifen aufgeklebt ist, wobei es an einem Ende bündig mit dem Ende des Plastikstreifens abschließt. Das andere, von dem Testpapier nicht bedeckte Ende des Plastikstreifens dient zur Handhabung des Testelementes (ähnlich einem Teststreifen). Zur Durchführung des Tests wird das Mekonium auf das untere Ende des Testpapiers aufgebracht und der Streifen derartig in ein enges, mit Wasser gefülltes Gefäß gestellt, daß das Wasser nicht mit dem Teil des Testpapiers in Kontakt kommt, der oberhalb der mit Mekonium behandelten Stelle liegt. Das in das Mekonium eindringende Wasser löst das Albumin und transportiert es in das Testpapier, welches einen Indikator enthält, der mit dem Albumin eine blaue Farbe bildet. Die Farbe wird in dem Testpapier chromatographiert und gelangt dadurch aus dem Bereich, in dem das Mekonium aufgetragen wurde, heraus, so daß sie sichtbar wird. Hinweise bezüglich der mit dem Gegenstand der vorliegenden Erfindung verbundenen besonderen Probleme und deren Lösung sind der Literaturstelle nicht zu entnehmen.

Es hat sich als grundsätzlich ausreichend erwiesen, wenn die Probe beispielsweise durch Aufstreichen mit einem Spatel auf das Probenfeld an der Oberfläche der Probenschicht aufgebracht wird. Besonders bevorzugt ist die Probenschicht im Bereich des Probenfeldes jedoch so offen strukturiert, daß die Probe zumindest teilweise darin eindringt. Dadurch wird eine verbesserte Eluation des Analyten erreicht.

Der Flüssigkeitsaustausch ermöglichende Kontakt zwischen Eluationsmittelauftragungsbereich und Probenschicht sowie von der Probenschicht zur Nachweisschicht kann auf verschiedene Weise, zum Beispiel in Form von die verschiedenen Bereiche verbindenden Spalten oder Röhren realisiert werden Besonders bevorzugt ist jedoch die Probenschicht Teil einer größeren Flüssigkeitstransportschicht, welche einerseits bis in den Eluationsmittelaufgabebereich und andererseits bis in den Eluationsmittelaufnahmebereich reicht. Eine derartige Ausführungsform ist besonders einfach herstellbar und besonders zuverlässig in der Funktion.

Überraschenderweise hat sich gezeigt, daß beiden erfindungsgemäßen Testkits nicht darauf geachtet werden muß, daß der Stuhl im frischen Zustand analysiert wird. Soweit - insbesondere bei sich über mehrere Tage erstreckenden Untersuchungen - der Stuhl längere Zeit gelagert wird, hat es sich sogar als zweckmäßig erwiesen, besondere Maßnahmen zu ergreifen, die das Eintrocknen des Stuhls fördern.

Vorzugsweise umfaßt der Probenauftragunsbereich mehrere voneinander getrennte Probenauftragsfelder, die in Flüssigkeitsaustausch zueinander stehen, wobei dies am günstigsten dadurch erreicht wird, daß nur eine Probenschicht verwendet wird, auf der die Probenfelder vorgesehen sind. In der Praxis hat sich gezeigt, daß drei nacheinander auf einer Schicht vorgesehene Stuhlproben ausreichend reproduzierbar eluiert werden können, sodaß auf der Nachweisschicht ein Signal (insbesondere ein Farbumschlag) erzeugt wird, sofern nur eine der Proben den Analyten enthält. Dadurch wird eine Integration des Analyseergebnisses über die drei Proben und damit eine Erhöhung der Nachweissicherheit erreicht.

Mit dem erfindungsgemäßen Testkit können verschiedene, in einem Eluationsmittel lösliche Bestandteile des Stuhls (Analyten) bestimmt werden. Insbesondere hat sich gezeigt, daß sich Hämoglobin damit als Indikator für okkultes Blut bestimmen läßt. Andere geeignete Analyten sind beispielsweise Elektrolyte, wasserlösliche Proteine und Enzyme (insbesondere Proteasen).

Als besonders geeignet hat sich der Testkit jedoch für die Bestimmung von Humanserumalbumin erwiesen. Im Rahmen der Erfindung wurde gefunden, daß Humanserumalbumin ausgezeichnet als Indikator für die Anwesenheit von okkultem Blut im Stuhl geeignet ist. Bei richtiger Einstellung der Nachweisschwelle (beispielsweise auf etwa 0,1% (v/w) Blut im Stuhl,wobei sich der geeignete Wert jedoch empirisch noch genauer festlegen läßt)ergibt sich, daß okkultes Blut im Stuhl mit Albumin sowohl spezifischer als auch empfindlicher festgestellt werden kann, als über Pseudo-Peroxidaseaktivität im Guajak-Test oder auch als in der immunologischen Hb-Bestimmung. Es ergeben sich also insgesamt sowohl weniger falsch-positive als auch weniger falsch-negative Befunde.

Der Testträger weist eine oder mehrere reagenzienhaltige Testschichten auf. Testträger haben üblicherweise eine Nachweisschicht, wobei am Ende der Reaktion in der Nachweisschicht eine signalproduzierende Komponente gebildet wird oder in diese hineinwandert. In diesem Sinne kommt grundsätzlich jedes meßtechnisch feststellbare Signal, beispielsweise die radioaktive Strahlung einer radioaktiven Markierungssubstanz, in Betracht. Die Erfindung richtet sich bevorzugt jedoch auf visuell oder mit einfachen reflexionsphotometrischen Geräten auswertbare Tests, bei denen das Signal in einem Farbumschlag besteht. Als Nachweisreagenzien kommen zahlreiche, in der klinischen Chemie für den jeweils zu bestimmenden Analyten gebräuchliche Testsysteme in Betracht.

Vorzugsweise wird ein immunologisches Nachweissystem verwendet. Es hat sich gezeigt, daß das Eluat so weitgehend frei von Stuhlbestandteilen ist, daß trotz der extrem einfachen Handhabung eine ausreichende Vorbereitung auch für die empfindlichen immunologischen Bestimmungen erreicht wird.

Insbesondere für immunologische Bestimmungsverfahren ist es vorteilhaft, daß der Testkit mindestens zwei voneinander getrennte Teile, nämlich eine Probensammeleinheit und eine Nachweiseinheit umfaßt. Die Nachweiseinheit kann getrennt hergestellt und verpackt und erst zum Zwecke der Auswertung vor Zugabe des Eluationsmittels in Flüssigkeitsaustausch ermöglichenden Kontakt mit der Probenschicht gebracht werden. Damit wird eine besonders kostengünstige Produktion und gute Lagerbeständigkeit, insbesondere der Nachweiseinheit, erreicht.

Die Erindung wird im folgenden anhand von in den Figuren schematisch dargestellten Ausführungsbeispielen näher erläutert. Es zeigen:

Fig. 1   eine Prinzipdarstellung eines Labormusters, welches die Erfindung noch nicht in vollem Umfang realisiert,

Fig. 2   die Probensammeleinheit eines erfindungsgemäßen Testkits in einer Explosionsdarstellung,

Fig. 3   die Probensammeleinheit nach Fig. 2 mit einer darin eingesetzten Nachweiseinheit im Querschnitt,

Fig. 4   eine alternative Ausführungsform einer Nachweiseinheit im Querschnitt.

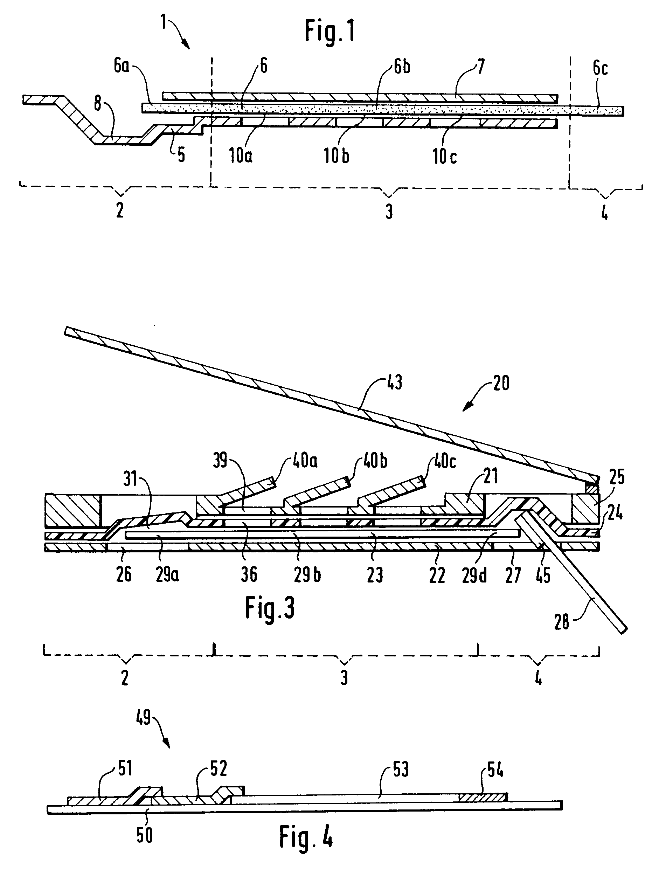

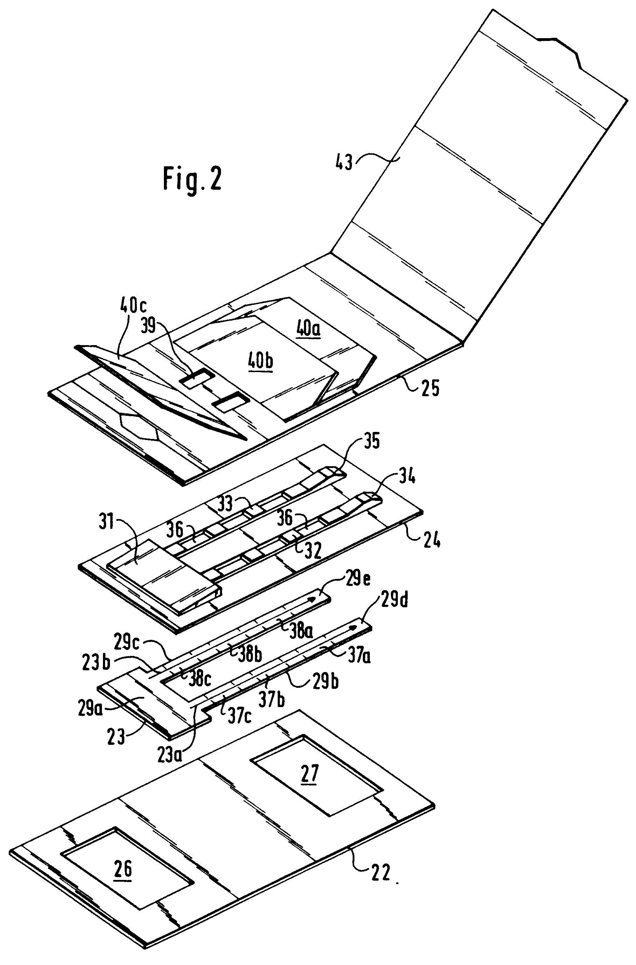

Das in Fig. 1 dargestellte Labormuster 1 läßt sich seiner Länge nach in einen Eluationsmittelaufgabebereich 2, einen Probenauftragsbereich 3 und einen Eluataufnahmebereich 4 unterteilen. Er besteht im wesentlichen aus einem Kunststoffgrundkörper 5, einer durchgehenden Flüssigkeitstransportschicht 6 und einer darüber angeordneten, beispielsweise aus Kunststoff bestehenden Abdeckschicht 7.

Im Eluationsmittelauftragsbereich 2 ist der Kunststoffgrundkörper 5 zu einer Eluationsmittelwanne 8 ausgeformt. Im Probenaufgabebereich 3 weist er drei Durchbrechungen auf. Die Durchbrechungen umrahmen Probenfelder 10a, 10b,10c für drei verschiedene Stuhlproben.

Die Flüssigkeitstransportschicht 6 ist im dargestellten Fall eine einzige durchgehende Schicht. Im Eluationsmittelaufgabebereich 2 bildet sie eine Ansaugschicht 6a für ein in die Eluationsmittelwanne 8 eingegebenes Eluationsmittel. Im Probenauftragsbereich 3 bildet sie eine Probenschicht 6b mit den Probenfeldern 10a,10b,10c an ihrer Oberfläche. Im Eluataufnahmebereich 4 bildet sie eine Eluataufnahmeschicht 6c.

Zum Auftragen der Probe wird das Labormuster mit den Probefeldern nach oben (also umgekehrt wie dargestellt) gehalten. Die zu untersuchenden Stuhlproben werden auf die Probenfelder 10a,10b,10c derart aufgetragen, daß sie jeweils zumindest die Oberfläche der Probenschicht 6b bedecken, vorzugsweise in diese teilweise eindringen. Die Probe wird danach eintrocknen gelassen.

Zur Auswertung wird ein Eluationsmittel,vorzugsweise Wasser oder eine wässrige Lösung mit Hilfsreagenzien (beispielsweise Netzmittel und Puffer) in die Eluationsmittelwanne 8 gebracht oder unmittelbar auf die Ansaugschicht 6a aufgetropft. Die Ansaugschicht 6a saugt das Eluationsmittel auf, so daß dieses aus dem Eluationsmittelauftragsbereich 2 in den Probenauftragsbereich 3 und - stets durch Kapillarkräfte getrieben - weiter in den Eluataufnahmebereich 4 gelangt. Im Probenauftragsbereich 3 löst das Eluationsmittel den löslichen Analyten aus der Probe und transportiert ihn mit in den Eluataufnahmebereich 4. Dies geschieht für praktische Zwecke ausreichend reproduzierbar, während gleichzeitg feste Stuhlbestandteile, die den Nachweis stören könnten, im Probenauftragsbereich 3 zurückbleiben. Es ergibt sich somit eine außerordentlich einfache Probenaufbereitung für die eigentliche Nachweisreaktion, welche bei dem Labormuster in der Weise durchgeführt werden kann, daß man die Eluataufnahmeschicht 6c abtrennt das Eluat herunterwäscht und in flüssiger Phase analysiert. Alternativ kann die Eluataufnahmeschicht 6c zugleich als Testschicht dienen.

Selbstverständlich kann ein Teil der für den Nachweis notwendigen Reagenzien schon in dem Eluationsmittel oder in der Flüssigkeitstransportschicht vorhanden sein, soweit die Wirkung dieser Reagenzien durch die Gegenwart des Stuhls nicht gestört wird.

Die Figuren 2 und 3 zeigen eine einfach herstellbare und zweckmäßige Ausführungsform eines erfindungsgemäßen Testkits 20, welcher insbesondere für den Nachweis von okkultem Blut im Stuhl im Rahmen der Krebsvorsorge geeignet ist und zu diesem Zweck insgesamt sechs an drei aufeinanderfolgenden Tagen gewonnenen Stuhlproben in besonders einfacher und genauer Art und Weise auswerten kann.

Die Probensammeleinheit 21 dieses Testkits besteht aus einem Basisteil 22, einem Einsatzteil 23, einem Kunststofformteil 24 und einem Abdeckteil 25. Auch in diesem Fall läßt sich der Testkit 20 in einem Eluationsmittelaufgabebereich 2, einen Probenauftragsbereich 3 und in einen Eluataufnahmebereich 4 unterteilen.

Das Basisteil 22 kann beispielsweise aus wasserfestem Karton hergestellt sein und weist zwei Öffnungen 26 und 27 auf, durch die Eluationsmittel zugegeben bzw. eine Nachweiseinheit 28 in die Probensammeleinheit 2l eingeführt werden kann.

Das Einsatzteil 23 enthält zwei durch die Pfeile 23a und 23b bezeichnete Flüssigkeitstransportstrecken. Sie werden im Eluationsmittelaufgabebereich von einer gemeinsamen Eluationsmittelansaugeschicht 29 a gebildet, welche im Probenauftragsbereich 3 in zwei getrennte Probenschichten 29b,29c übergeht. Die Probenschicht 29b,29c gehen ihrerseits in Eluataufnahmeschichten 29d,29e über.

Die dargestellte einstückige Ausführung des Einsatzteils 23 ist besonders bevorzugt, jedoch können auch getrennte, in Flüssigekeitsverbindung zueinanderstehende Schichten verwendet werden.

In dem Kunststofformteil 24 sind eine Eluationsmittelwanne 31, zwei Flüssigkeitstransportkanäle 32,33 und zwei Aufnahmevertiefungen 34,35 für jeweils eine Nachweiseinheit 28 ausgebildet. Die Flüssigkeitstransportkanäle 32,33 haben Öffnungen 36, durch welche Proben auf die Probenschichten 29b,29c aufgegeben werden können. Die Probenfelder 37 bzw. 38 auf den Probenschichten 29b,29c, die von den Öffnungen 36 umrahmt werden, sind in Fig. 2 der Deutlichkeit halber durch gestrichelte Linien markiert.

Das Abdeckteil 25 kann wiederum aus wasserfestem Karton bestehen. Es weist Öffnungen 39 auf, die mit den Öffnungen 36 der Flüssigkeitstransportkanäle 32,33 fluchten. Drei Klappen 40a,40b,40c dienen als Abdeckelemente, um die aufgetragene Probe geruchsdicht und hygienisch zu verschließen. Auf der Unterseite der Klappen ist eine Haftschicht angebracht, die ein zuverlässiges Verschließen der Klappen sicherstellt.

Andererseits ist die Verbindung zwischen den Teilen 22,23,24,25 bewußt nicht völlig dicht gestaltet, so daß Feuchtigkeit von der aufgetragenen Probe langsam entweichen kann, wobei diese eintrocknet.

Die gesamte Stuhlprobensammeleinheit läßt sich mit einer Verschlußkappe 43 zusätzlich verschließen. Zweckmäßigerweise dient die Innenseite der Verschlußklappe 43 zugleich als Träger für eine aufgedruckte Bedienungsanleitung.

Der Testkit gemäß den Figuren 2 und 3 wird in der Weise benutzt, daß zunächst die Klappe 40a geöffnet und zwei Proben von verschiedenen Stellen eines Stuhls auf die darunter liegenden Probenfelder 37a bzw. 38a aufgetragen werden. Danach wird die Klappe 40a verschlossen. In analoger Weise werden an den darauffolgenden Tagen jeweils zwei Proben von verschiedenen Stellen des Stuhls auf die unter den Klappen 40b bzw. 40c liegenden Probenfelder 37b, 38b bzw. 37c,38c aufgetragen und die Klappen 40b

5

EP 0 291 843 B1

bzw. 40c danach verschlossen.

Nachdem sämtliche Proben aufgetragen sind, wird die Verschlußklappe 43 geschlossen und die Probensammeleinheit wird zum Zweck der Analyse an einem der folgenden Tage zum Arzt gebracht.

Zur Analyse wird im Arztlabor die Probensammeleinheit 21 mit den Öffnungen 26 und 27 nach oben gehalten. Durch die Öffnung 27 wird in die Aufnahmevertiefungen 34 und 35 jeweils eine Nachweiseinheit in Form eines Testträgers eingesetzt. Der in Fig. 3 dargestellte Testträger hat eine einzige Testschicht 45.

Danach wird Eluationsmittel durch die Öffnung 26 in die Eluationsmittelwanne gegeben.

Aufgrund der in den Schichten 29a, 29b, 29d bzw. 29a, 29c, 29e wirkenden Kapillarkräfte fließt das Eluationsmittel entlang den Flüssigkeitstransportstrecken 23a, 23b bis in den Eluationsmittelaufnahmebereich 4, wobei es in der Probenauftragszone 3 den Analyten aus der Probe löst und mittransportiert.

Zur Analyse muß das Eluat von den Eluataufnahmeschichten 29d, 29e am Ende der Flüssigkeitstransportstrecke 23a,23b auf den Testträger 28 übergehen. Dies geschieht bevorzugt einfach dadurch, daß die Testschicht 45, wie in Fig. 3 dargestellt, mit dem Ende der Eluationsmittelaufnahmeschicht 29d bzw. 29e in Kontakt gebracht wird. Es kann jedoch auch eine spezielle Übertragungseinrichtung, beispielsweise ein zusätzliches Vlies, zur Übertragung des Eluats auf den Testträger 28 vorgesehen sein.

Die Testschicht 45 des Testträgers 28 kann sämtliche für die Analyse notwendigen Reagenzien einschließlich der ein Nach weissignal erzeugenden Komponente enthalten. Beispielsweise kann in ihr eine für den Analyten spezifische enzymatische Reaktion ablaufen, die direkt oder indirekt zu einer Farbänderung auf der Oberfläche des Testfelds führt. Derartige Reaktionen sind in zahlreichen Varianten bekannt und müssen hier nicht näher erläutert werden.

Der Testkit gemäß den Figuren 2 und 3 ist besonders für die Analyse mit Hilfe eines immunologischen Testsystems geeignet.

Auch in diesem Fall kann eine einzige Testschicht verwendet werden, wie weiter unten anhand von Beispiel 4 näher erläutert wird.

Besonders bevorzugt ist bei Verwendung eines immunologischen Testsystems für den Nachweis des Analyten jedoch ein Testträgeraufbau, wie er prinzipiell in Figur 4 dargestellt ist. Ein derartiger Testträger 49 besteht im wesentlichen aus einer Basisschicht 50 und mehreren darauf nebeneinander angeordneten Schichten 51,52,53,54 welche in Flüssigkeitskontakt zueinander stehen. Der Flüssigkeitskontakt kann dadurch erreicht werden, daß die Kanten aneinanderstoßen oder daß sich die Schichten gegenseitig geringfügig überlappen. Das Eluationsmittel erfüllt hier eine doppelte Funktion. Es dient sowohl der Probenvorbereitung in der Probensammeleinheit 21 als auch der Auswertung in dem Testträger 49.

Der Testträger 49 hat eine spezielle Übernahmeschicht 51, die in ihren Saugeigenschaften so abgestimmt ist, daß sie das Eluat von der Eluataufnahmeschicht 29d besonders gut reproduzierbar aufsaugt.

Besonders bevorzugt ist der Testträger für Analysen ausgebildet, die dem sogenannten IEMA-Prinzip folgen. In diesem Fall enthält er nacheinander eine Konjugatschicht 52, mit einem für den Analyten spezifischen, mit einem Markierungsenzym markierten ersten Bindungspartner in löslicher Form, eine Festphasenbindungsschicht 53 mit dem Analyt oder einem Analyt-Analogen und eine Signalbildungsschicht 54, mit einer in Abhängigkeit von der Anwesenheit des Markierungsenzyms signalbildenden Komponente.

Ist beispielsweise der Analyt ein Antigen, so befindet sich auf der Konjugatschicht 52 ein enzymmarkierter Antikörper für dieses Antigen. Die Festphasenbindungsschicht 53 enthält das Antigen oder ein Antigenanalogon, also eine Substanz, welche ihrerseits mit dem Antikörper aus der Schicht 51 spezifisch bindungsfähig ist, in trägerfixierter Form. Die Signalbildungsschicht 54 kann in diesem Fall ein farbbildendes Substrat des Markierungsenzyms enthalten, welches in Gegenwart des Enzyms eine mit einer Farbänderung verbundene Umwandlung erfährt.

Setzt man einen Testträger 49 gemäß Figur 4 in eine Probensammeleinheit 21 gemäß Figur 3 ein, so tritt das Eluat zunächst in die Übernahmeschicht 51 über und wandert dann, durch Kapillarkräfte getrieben, langsam durch die Schichten 52,53,54 Dabei wird das Konjugat in der Schicht 52 gelöst, wobei der darauf im Überschuß vorhandene Antikörper mit dem Antigen bindet. Sowohl die gebildeten Komplexe als auch überschüssige Antikörper wandern weiter auf die Schicht 53. Dort bindet der Antikörper-Überschuß an das trägerfixierte Antigen, während die Komplexe beweglich bleiben und in die Signalbildungsschicht 53 gelangen können. Der dort stattfindende Farbumschlag entspricht der Enzymkonzentration und damit der Konzentration des Analyt-Antigens.

Ein derartiger Testablauf ist im Prinzip bekannt. Nähere Einzelheiten sind beispielsweise in der deutschen Patentanmeldung 36 38 654.5 und den darin zitierten Druckschriften beschrieben. Wie dort im einzelnen dargelegt wird, ist es im allgemeinen wichtig, immunchemische Testträger so aufzubauen, daß die verschiedenen Analyseschritte sauber, zeitlich getrennt voneinander ablaufen. Im Rahmen der vorliegenden Erfindung hat sich jedoch ergeben, daß für Zwecke der Stuhlanalyse mit einem erfindungsgemäßen Testkit ein einfacher Testträgeraufbau gemäß Figur 4, bei dem das Eluat ohne besondere Steuermittel von

6

einer Schicht in die nächste übertreten kann, ausreichend ist.

Die folgenden Beispiele dienen der weiteren Erläuterung der Erfindung:

Beispiel 1:

Immunchemische Bestimmung von Humanblutbestandteilen in Stuhlproben durch Analyse auf Humanserumalbumin(HSA).

Auf die Probenfelder 10 eines Labormusters gemäß Figur 1 wurden jeweils drei Stuhlproben aufgebracht und über Nacht eingetrocknet. Die Probenschicht bestand aus Papier VS 532 der Fa. Binzer, Hatzfeld, BRD mit folgender Zusammensetzung: 20% Linters, 30% Zellwolle, 10% Kuralon, 40% Polyamid.

Es wurden 150 $\mu$l Eluationsmittel (PBS-Puffer: 150 mM NaCl; 50 mM KPi; pH 7,4) verwendet.

Sobald die Flüssigkeitsfront das Ende der Eluataufnahmeschicht 6c erreicht hatte, wurde diese abgeschnitten und mit einem Enzymimmuntest (EIA) auf HSA untersucht.

Der HSA-Test lief folgendermaßen ab:
- die abgeschnittene Eluataufnahmeschicht wurde 10 Minuten in 500 $\mu$l PBS (Phosphate Buffer Saline) eluiert,
- 250 $\mu$l des Eluat wurden 60 Minuten in Mikrotiterplatten, beladen mit 10 $\mu$g Anti⟨HSA⟩ -IgG/well, inkubiert.
- die wells wurden 3 mal mit PBS + 0,05 % (w/v) Tween 20® (einem nicht-ionischen Detergents der Atlas-Chemie,Essen, BRD), gewaschen.
- Anschließend wurden den wells je 300 $\mu$l Anti HSA - Fab-$\beta$-Galaktosidase-Konjugat zugesetzt und 30 Minuten geschüttelt.
- Die wells wurden, wie oben beschrieben, gewaschen
- Nach Zugabe von je 300 $\mu$l Chlorphenolrot-Galactosid (0,25 mM in 10 mM KPi; 25 mM NaCl; 5 mM MgCl2; pH 7,6) wurden die Platten 10 Minuten geschüttelt und anschließend das umgesetzte Substrat bei 577 nm in einem Mikrotiterplattenphotometer vermessen.
- Aus einer mitgeführten Standardverdünnungsreihe wurden die Absolutwerte ermittelt.

Die folgende Tabelle 1 zeigt die Ergebnisse. Dabei ist in der oberen Zeile angegeben, in welche der Probenfelder 10 jeweils eine Stuhlprobe mit 0,1% (v/w) Blutzumischung eingegeben wurde. In der Zweiten Zeile ist die bei der Messung bestimmte Menge an HSA angegeben.

## Tabelle 1

| | Position der Stuhlprobe(n) mit 0,1% Blutzumischung | | | | | | | |
|---|---|---|---|---|---|---|---|---|
| | 1 | 2 | 3 | 1+2 | 2+3 | 1+3 | 1+2+3 | Kontrolle |
| ng HSA | 60 | 80 | 60 | 110 | 120 | 100 | 170 | <5 |

Bei dem Kontrollexperiment wurden 3 Stuhlproben ohne Blutzumischung eingesetzt.

Die Ergebnisse zeigen, daß die ermittelten HSA-Werte weitgehend unabhängig von der Position der bluthaltigen Stuhlproben und proportional zu der Gesamtzahl der bluthaltigen Proben sind. Es wird also eine zuverlässige Analyse, insbesondere ein zuverlässiger Nachweis von okkultem Blut im Stuhl erreicht.

Beispiel 2:

Nachweis von okkultem Blut im Stuhl auf Guajakbasis

Stuhlproben wurden, wie in Beispiel 1, in ein Labormuster gemäß Figur 1 eingebracht und eingetrocknet. Als Eulataufnahmeschicht wurde ein Guajak-imprägniertes Papier aus einem handelsüblichen Stuhlbluttest (hemoFec der Boehringer Mannheim GmbH, Mannheim, BRD) so eingebaut, daß ein Flüssigkeitsaustausch mit der Probenschicht 6b möglich war. Wie zuvor wurde der Analyt aus den Proben eluiert und das mit dem Eluat angefeuchtete Guajak-imprägnierte Papier wurde mit der üblichen Wasserstoffperoxid enthaltenden Entwicklerlösung beträufelt und der Test visuell ausgewertet.

Die Ergebnisse sind in Tabelle 2 dargestellt, wobei die verschiedenen Zeilen die Ergebnisse mit den jeweils am Anfang der Zeile angegebenen Blutkonzentrationen im Stuhl angeben. Die letzte Spalte zeigt die Ergebnisse mit einem handelsüblichen hemoFec-Test.

Tabelle  2

| %(v/w) Blut/ Stuhl | Position der Stuhlprobe(n) mit Blutzumischung | | | | | | | |
|---|---|---|---|---|---|---|---|---|
| | 1 | 2 | 3 | 1+2 | 2+3 | 1+3 | 1+2+3 | hemoFec |
| 0,1 % | - | - | - | - | - | - | - | - |
| 0,6 % | - | - | - | + | + | + | + | - |
| 1,0 % | - | +- | +- | + | + | + | ++ | +- |
| 2,0 % | + | + | + | ++ | ++ | ++ | ++ | .+ |

+ bedeutet eine klar positive Anzeige;

- bedeutet eine klar negative Anzeige;

+- bedeutet zweifelhafte Werte;

++ bedeutet eine besonders deutliche, eine hohe Blutkonzentration anzeigende Verfärbung;

Man erkennt, daß die Empfindlichkeit bei nur einer bluthaltigen Probe im Test gegenüber dem handelsüblichen hemoFec-Test nicht leidet. Damit zeigt sich, daß die mit dem Guajak nachgewiesenen peroxidatischen Blutbestandteile ausreichend quantitativ eluiert werden.

Von besonderer Bedeutung ist, daß bei Anwesenheit von mindestens zwei bluthaltigen Proben die Empfindlichkeit des Tests wesentlich erhöht wird, so daß auch bei Verwendung eines Guajak-Nachweissystems falsch-negative Befunde vermieden werden.

Beispiel 3

Bestimmung von HSA bzw. okkultem Blut mit einem Testkit entsprechend den Figuren 2 und 3 unter Verwendung eines Testträgers nach Figur 4.

Die Übernahmeschicht 51 hat eine Fläche von 6 x 6 mm und besteht aus unbehandeltem Papier VS 532, die Firma Binzer, Hatzfeld, BRD verwendet.

Die Konjugatschicht 52 ist 10 x 6 mm groß und besteht aus multifilem Polyestergewebe 2F 777 der Schweizer Seidengaze Fabrik, Thal, Schweiz. Das Gewebe ist getränkt mit einer Lösung von fab-$\beta$-Galactosidase-Konjugat HSA.

Die Festphasenbindungsschicht 53 und die Signalbildungsschicht 54 bestehen insgesamt aus einer porösen Nylonmembran "Biodyne BNRG 0,2 $\mu$m "die von der Firma Pall, Dreieich, BRD vertrieben wird.

Auf der Festphasenbindungsschicht 53 ist HSA fixiert. Dazu wird die Membran über Nacht in eine Lösung mit einer HSA-Konzentration von 10 mg/ml HSA, die mit 50 mM KPi (Kaliumphosphat) pH 7,4 und 150 M mol NaCl gepuffert wird,eingelegt. Am nächsten Tag wird sie dreimal mit dem gleichen Puffer, aber ohne HSA, gewaschen und getrocknet.

Die Signalbildungsschicht 54 ist zusätzlich mit Chlorphenolrod-Galaktosid (CPRG) imprägniert.

Der so aufgebaute Testträger wird in der im Zusammenhang mit den Figuren 2 - 4 beschriebenen Art und Weise verwendet. In dem Eluat enthaltenes HSA reagiert in der Konjugatschicht 52 mit dem dort imprägnierten Anti-HSA-$\beta$-Galaktosedase-Konjugat. Nur die so gebildeten Komplexe sind in der Lage, die Festphasenbindungsschicht 53 zu überwandern und das in der Signalbildungsschicht 54 enthaltene CPRG umzusetzen.

Der gesamte Durchlauf des Eluationsmittels durch die Probensammeleinheit 21 und den Testträger 49 dauert ca. 5 - 7 Minuten. Innerhalb von 10 - 15 Minuten ändert sich die Farbe des Substrats, so daß viusell ausgewertet werden kann. Die Gegenwart von HSA wird durch eine Rot-Verfärbung angezeigt.

Reihenversuche mit derartigen Testkits haben ergeben, daß bei einer eingestellten Nachweisgrenze von 0,5% Blut im Stuhl überraschenderweise nicht nur weniger falsch-negative, sondern auch weniger falsch-positive Befunde festgestellt wurden. Das letztere ist besonders überraschend, weil bei den bisher verwendeten Testprinzipien stets höhere Nachweisgrenzen eingestellt wurden, um zu viele falsch-positive Befunde zu vermeiden.

Beispiel 4

Nachweis von HSA mit einem Sandwich-EIA

Es wurde ein Testträger verwendet, welcher entsprechend Figur 3 ein einziges Testfeld 45 hatte. Dieses bestand aus einer Nylonmembran (Pall Biodyne 1,2 $\mu$m, welche (durch Inkubation über Nacht in 10 mg Antikörper/ml 50 mM KPi/150 mM NaCl, pH7,4 und dreimaliges Waschen in 50 mM KPi pH7,4/150 mM NaCl) adsorptiv mit Anti ⟨HumanHämoglobin⟩ - IgG (polyklonal) beladen wurde.

Der mit dem Eluat benetzte Testträger wurde entnommen und unter fließendem Leitungswasser im Flüssigkeitsüberschuß gewaschen. Anschließend wurde er in einen Waschpuffer mit

150 mM NaCl
50 mM KPi, pH 7,4
0,05 % Tween 20®
1 mU/ml Anti ⟨hHB⟩ -$\beta$-Galactosidase-Konjugat

ca. 1 Minute eingestellt und darin mehrmals kurz gerührt. Danach wurde das Testfeld erneut unter fließendem Wasser gewaschen und direkt in eine CPRG-haltige Substratlösung eingestellt. Eine Farbreaktion auf der Membran zeigte die Anwesenheit von Blut in Stuhlproben zunverlässig an.

**Patentansprüche**

**1.** Testkit (20) zur Bestimmung eines Analyten in einer pastösen, feste Bestandteile enthaltenden Probe, insbesondere im Stuhl, welcher Testkit folgende Komponenten aufweist:

a) eine Probensammeleinheit (21) mit einem zwischen einem Basisteil (22) und einem Abdeckteil (25) angeordneten Einsatzteil (23), welche in einem Probenauftragsbereichh (3) eine Probenschicht (29b,29c) aufweist, die Bestandteil des Einsatzteils (23) ist und auf der ein Probenfeld (37,38) vorgesehen ist, welches durch eine das Probenfeld (37,38) umrahmende Öffnung (39) des Abdeckteils (25) zugänglich ist, wobei

aa) die Probenschicht (29b,29c) aus einem Material besteht, welches kapillaraktiv ist, so daß eine Flüssigkeit durch Kapillarwirkung darin transportiert wird,

ab) mit der Probenschicht (29b,29c) einerseits ein Eluationsmittelaufgabebereich (2) in Flüssigkeitsaustausch ermöglichender Verbindung steht, so daß ein dort aufgegebenes Eluationsmittel in die Probenschicht eindringt und einen in der Probe enthaltenen in dem Eluationsmittel löslichen Analyten eluiert und

ac) mit der Probenschicht (29b,29c) andererseits ein Eluationsmittelaufnahmebereich (4) in Flüssigkeitsaustausch ermöglichender Verbindung steht, von dem das Eluationsmittel mit dem eluierten Analyten aufgenommen wird

und

b) eine von der Probensammeleinheit (21) getrennte Nachweiseinheit in Form eines Testträgers (49) mit mindestens einer Testschicht (51-54), wobei die mindestens eine Testschicht (51-54) Reagenzien enthält, die mit dem Analyten reagieren und eine ein Nachweissignal erzeugende Komponente einschließen,

wobei der Eluataufnahmebereich (4) der Probensammeleinheit (21) eine Übertragungseinrichtung zur Übertragung des Eluats auf die Nachweiseinheit einschließt.

EP 0 291 843 B1

**2.** Testkit nach Anspruch 1, bei welchem die Probenschicht (29b,29c) der Probensammeleinheit (21) so offen strukturiert ist, daß die Probe beim Auftragen zumindest teilweise darin eindringt.

**3.** Testkit nach Anspruch 1, bei welchem die Probenschicht (29b,29c) der Probensammeleinheit (21) mit einem Abdeckelement (40a,b,c) derart abdeckbar ist, daß eine darauf aufgetragene Probe geruchsdicht und hygienisch verschlossen werden kann, wobei jedoch Feuchtigkeit von der Probe entweichen kann, so daß die Probe eintrocknet.

**4.** Testkit nach Anspruch 1, bei welchem der Probenauftragsbereich (3) der Probensammeleinheit (21) mehrere voneinander getrennte Probenfelder (10a,10b,10c) umfaßt, welche in Flüssigkeitsaustausch ermöglichender Verbindung miteinander stehen.

**5.** Testkit nach Anspruch 4, bei welchem der Probenauftragsbereich (3) der Probensammeleinheit (21) zwei jeweils eine Probenschicht (29b,29c) enthaltende Flüssigkeitstransportstrecken (23a,23b) aufweist, deren jede drei Probenfelder (37a,37b,37c; 38a,38b,38c) einschließt.

**6.** Testkit nach Anspruch 1, bei welchem die Nachweiseinheit ein immunologisches Testsystem zum Nachweis des Analyten einschließt.

**7.** Testkit nach Anspruch 6, bei welchem der Analyt Humanserumalbumin ist.

**8.** Testkit nach Anspruch 1, bei welchem der Testträger (49) mehrere im wesentlichen nebeneinander angeordnete, in Flüssigkeitskontakt zueinander stehende Schichten (51-54) umfaßt, so daß die Eluationsmittel mit dem Analyten die Schichten nacheinander durchwandert.

**9.** Testkit nach Anspruch 8, bei welchem der Testträger (49) in Richtung der Eluationsmittelströmung nacheinander eine Konjugatschicht (52) mit einem für den Analyten spezifischen, mit einer Markierung markierten ersten Bindungspartner in löslicher Form, eine Festphasenbindungsschicht (53) mit dem Analyt oder einem Analyt-Analogen in trägerfixierter Form und eine Signalbildungsschicht (54) mit einer in Abhängigkeit von der Anwesenheit der Markierung ein Nachweissignal erzeugender Komponente einschließt.

**10.** Verfahren zur Bestimmung eines Analyten in einer pastösen Probe, insbesondere im Stuhl, mit einem Testkit nach einem der vorhergehenden Ansprüche, bei welchem man eine Probe auf die Probenschicht aufträgt, danach ein Eluationsmittel dem Eluationsmittelauftragsbereich zuführt, abwartet, bis das Eluationsmittel die Probenschicht durchströmt hat und mit dem eluierten Analyten in den Eluataufnahmebereich gelangt ist, die mindestens eine Testschicht des Testträgers mit dem Eluat in dem Eluataufnahmebereich in Kontakt bringt und nach Bildung des Nachweissignals dieses auswertet.

**11.** Verfahren nach Anspruch 10, bei welchem man die Probe nach dem Auftragen und vor Zugabe des Eluationsmittels eintrocknen läßt.

**Claims**

**1.** Test kit for the determination of an analyte in a pasty sample containing solid components, especially in faeces, which test kit has the following components:
a) a sample collection unit (21) with an insert part (23), arranged between a base part (22) and a covering part (25), which, in a sample application region (3), has a sample layer (29b, 29c) which is a part of the insert part (23) and on which is provided a sample field (37, 38) which is accessible through an opening (39) of the covering part (25) surrounding the sample field (37, 38), wherein
aa) the sample layer (29b, 29c) consists of a material which is capillary-active so that a liquid is transported therein by capillary action,
ab) with the sample layer (29b, 29c) on the one hand there stands in connection an elution agent application region (2) making possible a liquid exchange so that an elution agent applied there penetrates into the sample layer and elutes an analyte contained in the sample and soluble in the elution agent and
ac) with the sample layer (29b, 29c) on the other hand there stands in connection an elution agent take-up region (4) making possible liquid exchange from which the elution agent is taken up with

10

EP 0 291 843 B1

the eluted analyte and

b) a detection unit, separate from the sample collection unit (21), in the form of a test carrier (49) with at least one test layer (51 - 54), said at least one test layer (51 - 54) containing reagents which react with the analyte and include a detection signal-producing component,

wherein the eluate take-up region (4) of the sample collection unit (21) includes a transmission device for the transmission of the eluate to the detection unit.

2. Test kit according to claim 1, in which the sample layer (29b, 29c) is so open-structured that the sample, upon application, penetrates therein at least partly.

3. Test kit according to claim 1, in which the sample layer (29b, 29c ) is coverable with a covering element (40a, b, c) in such a manner that a sample placed thereon can be closed free of odour and hygienically, whereby, however, moisture can escape from the sample so that the sample dries out.

4. Test kit according to claim 1, in which the sample application region (3) of the sample collection unit (21) includes several sample fields (10a, 10b, 10c) separate from one another which are in connection with one another permitting liquid exchange.

5. Test kit according to claim 4, in which the sample application region (3) of the sample collection unit (21) includes two liquid transport paths (23a, 23b) each containing a sample layer (29b, 29c), each of which includes three sample fields (37a, 37b, 37c; 38a, 38b, 38c).

6. Test kit according to claim 1, in which the analysis unit includes an immunological test system for the detection of the analyte.

7. Test kit according to claim 6, in which the analyte is human serum albumin.

8. Test kit according to claim 1, in which the test carrier (49) includes several layers (51 - 54) arranged substantially side by side in liquid contact with one another so that the elution agent with the analyte migrates successively through the layers.

9. Test kit according to claim 8, in which the test carrier (49), in the direction of the elution agent flow, successively includes a conjugate layer (52) with a first binding component in soluble form specific for the analyte labelled with a labelling, a solid phase binding layer (53) with the analyte or an analyte analogue in carrier-fixed form and a signal-forming layer (54) with the component producing a detection signal dependent upon the presence of the labelling.

10. Process for the determination of an analyte in a pasty sample, especially in faeces, with a test kit according to one of the preceding claims, in which one applies a sample to the sample layer, thereafter supplies an elution agent to the elution agent application region, waits until the elution agent has flowed through the sample layer and has passed with the eluted analyte into the eluate take-up region, brings at least one test layer of the test carrier into contact with the eluate in the eluate take-up region and, after formation of the detection signal, evaluates this.

11. Process according to claim 10, in which, after the application and before application of the elution agent, one allows the sample to dry.

**Revendications**

1. Kit de test (20) pour la détermination d'un analyte dans un échantillon pâteux, contenant des constituants solides, en particulier des selles, ledit kit de test comprenant les composants suivants :

a) une unité collecteur d'échantillon (21) comprenant une partie de garniture (23) disposée entre une partie de base (22) et une partie de recouvrement (25), qui présente, dans une zone d'application d'échantillon (3), une couche d'échantillon (29b,29c) qui fait partie de la partie de garniture (23) et sur laquelle est prévue une zone d'échantillon (37,38), qui est accessible par l'intermédiaire d'un orifice (39) de la partie de recouvrement (25), entourant la zone d'échantillon (37,38), unité dans laquelle

EP 0 291 843 B1

aa) la couche d'échantillon (29b,29c) est constituée par un matériau qui présente une activité capillaire, de sorte qu'il peut transporter un fluide par capillarité,

ab) la couche d'échantillon (29b,29c) est d'une part en relation d'échange de liquide avec une zone d'application d'éluant (2) de sorte qu'un éluant qui y est déposé pénètre dans la couche d'échantillon et élue un analyte contenu dans l'échantillon et soluble dans l'éluant, et

ac) la couche d'échantillon (29b,29c) est d'autre part en relation d'échange de liquide avec une zone d'absorption d'éluant (4), à partir de laquelle l'éluant contenant l'analyte élué est absorbé, et

b) une unité de détection séparée de l'unité collecteur d'échantillon (21), ayant la forme d'un support de test (49) avec au moins une couche de test (51-54), ladite au moins une couche de test (51-54) contenant des réactifs qui réagissent avec l'analyte et comprennent un composant produisant un signal détectable,

la zone d'absorption d'éluat (4) de l'unité collecteur d'échantillon (21) comprenant un dispositif de transfert pour transférer l'éluat dans l'unité de détection.

2. Kit de test selon la revendication 1, dans lequel la couche d'échantillon (29b,29c) de l'unité collecteur d'échantillon (21) présente une structure ouverte telle que l'échantillon y pénètre au moins partiellement lors de son application.

3. Kit de test selon la revendication 1, dans lequel la couche d'échantillon (29b,29c) de l'unité collecteur d'échantillon (21) peut être recouverte par un élément de recouvrement (40a,b,c) de telle façon qu'un échantillon déposé sur elle puisse être enfermé de façon hygiénique et sans laisser passer les odeurs, l'humidité de l'échantillon pouvant toutefois s'éliminer et l'échantillon pouvant ainsi sécher.

4. Kit de test selon la revendication 1, dans lequel la zone d'application d'échantillon (3) de l'unité collecteur d'échantillon (21) comprend plusieurs zones d'échantillon séparées (10a, 10b, 10c) qui sont en relation mutuelle d'échange de liquide.

5. Kit de test selon la revendication 4, dans lequel la zone d'application d'échantillon (3) de l'unité collecteur d'échantillon (21) présente deux voies de transport de liquide (23a,23b) contenant chacune une couche d'échantillon (29b,29c), dont chacune comprend trois zones d'échantillon (37a,37b,37c ; 38a,38b,38c).

6. Kit de test selon la revendication 1, dans lequel l'unité de détection comprend un système de test immunologique pour la détection de l'analyte.

7. Kit de test selon la revendication 6, dans lequel l'analyte est de l'albumine de sérum humain.

8. Kit de test selon la revendication 1, dans lequel le support de test (49) comprend plusieurs couches (51-54) essentiellement contigües, en contact mutuel d'échange de liquide, de sorte que l'éluant contenant l'analyte traverse les couches les unes après les autres.

9. Kit de test selon la revendication 8, dans lequel le support de test (49) comprend, dans le sens de l'écoulement de l'éluant, successivement une couche de conjugué (52) avec un premier partenaire de liaison spécifique de l'analyte, marqué avec un marqueur, sous forme soluble, une couche de fixation de phase solide (53) avec l'analyte ou un analogue d'analyte sous forme fixée à un support, et une couche de formation de signal (54) avec un composant produisant un signal détectable en fonction de la présence du marqueur.

10. Procédé de détermination d'un analyte dans un échantillon pâteux, en particulier des selles, à l'aide d'un kit de test selon l'une quelconque des revendications précédentes, dans lequel on applique un échantillon sur la couche d'échantillon, ensuite on dépose l'éluant sur la zone d'application d'éluant, on attend que l'éluant ait traversé la couche d'échantillon et pénètre, avec l'analyte élué, dans la zone d'absorption d'éluat, on met en contact ladite au moins une couche de test du support de test avec l'éluat dans la zone d'absorption d'éluat et après formation du signal détectable, on évalue celui-ci.

11. Procédé selon la revendication 10, dans lequel on laisse l'échantillon sécher après son application et avant l'adjonction de l'éluant.

12

Fig.1

6a    6    6b    7    6c

8

5    10a    10b    10c

2    3    4

43    20

39    40a    40b    40c

31    21    25

24

26    29a    36    29b    23    22    29d    27    45

28

Fig.3

2    3    4

49

51    52    53    54

50

Fig. 4

# Fig.2